# FASCICULE DE BREVET EUROPEEN

(11) **EP 4 433 378 B1**
(45) Date de publication et mention de la délivrance du brevet: **01.10.2025**
(21) Numéro de dépôt: 22757608.9
(22) Date de dépôt: 24.08.2022
(51) Int. Cl.: B65D 51/00

(54) **CAPUCHON DE FERMETURE POUR UN DISPOSITIF MEDICAL, ADAPTE A UNE PREHENSION MAGNETIQUE**
VERSCHLUSSKAPPE FÜR EIN MEDIZINISCHES GERÄT MIT EIGNUNG FÜR MAGNETISCHEN GRIFF
CLOSURE CAP FOR A MEDICAL DEVICE, SAID CAP BEING SUITABLE FOR MAGNETIC GRIP

(30) Priorité: 17.11.2021 FR 2112141
(43) Date de publication de la demande: 25.09.2024
(73) Titulaire: A. Raymond et Cie, 38000 Grenoble (FR)
(72) Inventeur: SIRCOULOMB, Pascal, 38560 Jarrie (FR); REY, Gaëtan, 38500 Voiron (FR); CLAVEL, Maxime, 38450 Vif (FR); PELLET, Stéphanie, 38360 Sassenage (FR); YONNET, Jean-Paul, 38240 Meylan (FR); DE BORTOLI, Marc, 64230 Artiguelouve (FR); MALAQUIN, Linda, 38410 Saint Martin d'Uriage (FR); TENAUD, Philippe, 38190 La Combe de Lancey (FR)
(74) Mandataire: IP Trust
(86) Numéro de dépôt international: PCT/EP2022/073601
(87) Numéro de publication internationale: WO 2023/088587

(56) Documents cités:
- FR-A1- 2 598 137

## Description

### DOMAINE DE L'INVENTION

La présente invention concerne le domaine des dispositifs médicaux et en particulier le domaine des procédés industriels nécessitant la manipulation des composants de dispositifs médicaux, jusqu'à leur conditionnement pour l'utilisateur final. Elle concerne en particulier un capuchon de fermeture pour un dispositif médical, ledit capuchon étant adapté à une préhension magnétique.

### ARRIERE PLAN TECHNOLOGIQUE DE L'INVENTION

Un dispositif médical peut être composé d'un récipient (par exemple un flacon), destiné à être rempli d'un produit pharmaceutique ou chimique, et d'un capuchon de fermeture, destiné à clore ledit récipient, de façon hermétique et sécurisée (c'est-à-dire assurant un verrouillage inviolable jusqu'à l'utilisation finale). Le document FR2598137 montre un exemple d'un tel capuchon.

Le flacon d'un dispositif médical, fourni à un laboratoire, entre habituellement dans une chaine industrielle de remplissage avant d'être clos et verrouillé par le capuchon de fermeture. Les flacons (respectivement les capuchons) sont livrés par batch, dans des contenants dédiés, stériles, dans lesquels les flacons (respectivement les capuchons) sont maintenus classiquement par des plateaux, comme cela est par exemple le cas dans le document EP2753550.

La manipulation des flacons et des capuchons, dans la chaine de remplissage, est opérée par préhension mécanique (pince) ou plus souvent par aspiration (buse aspirante) car cette dernière option est moins susceptible de rayer ou endommager les éléments.

Les systèmes d'aspiration, en particulier pour la manipulation des capuchons, présentent néanmoins l'inconvénient d'aspirer de potentielles vapeurs chimiques, voire toxiques, relâchées par le produit introduit dans les flacons, avant leur fermeture : il existe donc un risque que ces vapeurs soient ensuite relâchées de manière non contrôlée en sortie desdits systèmes d'aspiration.

### OBJET DE L'INVENTION

La présente invention vise à remédier, en tout ou partie, à l'inconvénient précité. L'invention concerne un capuchon de fermeture pour un dispositif médical de type flacon, permettant la fermeture hermétique du flacon rempli de produit pharmaceutique ou chimique, et son verrouillage, le capuchon de fermeture étant adapté à une préhension magnétique, notamment dans les chaines industrielles de remplissage en produit pharmaceutique ou chimique.

### BREVE DESCRIPTION DE L'INVENTION

L'invention concerne un capuchon de fermeture pour un dispositif médical de type flacon, comprenant :
- un bouchon élastomère destiné à être inséré dans un col d'un flacon après son remplissage avec un produit pharmaceutique ou chimique,
- une coiffe de verrouillage plastique entourant le bouchon et destinée à se verrouiller sous une collerette du flacon lorsque le bouchon est inséré dans le col, la coiffe de verrouillage comprenant, au niveau d'une partie supérieure, une capsule,
- un matériau magnétique intégré en tout ou partie dans la capsule, et dans lequel :
   - la capsule est formée d'une matière plastique et une pièce, formée dans le matériau magnétique, est complètement ou partiellement encapsulée dans la matière plastique de ladite capsule, ou
   - la capsule est formée d'une matière plastique chargée en particules constituées du matériau magnétique.

Selon d'autres caractéristiques avantageuses et non limitatives de l'invention, prises seules ou selon toute combinaison techniquement réalisable :
- le matériau magnétique forme un aimant permanent, en particulier un aimant néodyme-fer-bore NdFeB ou un aimant en ferrite ;
- le matériau magnétique est ferromagnétique, choisi en particulier parmi le fer, le cobalt, le nickel, ou des alliages les contenant ;
- le matériau magnétique est ferrimagnétique, choisi en particulier parmi les ferrites grenats ou spinelles ;
- la coiffe de verrouillage comprend, au niveau d'une partie supérieure, une capsule, et le matériau magnétique est solidaire de ou intégré en tout ou partie dans ladite capsule ;
- la capsule est formée d'une matière plastique, et une pièce formée dans le matériau magnétique est collée sur une face de ladite capsule ou insérée en force dans un logement aménagé sur une face de ladite capsule ;
- la capsule est formée d'une matière plastique, et une pièce, formée dans le matériau magnétique, est encapsulée dans la matière plastique de ladite capsule ;
- la capsule est formée d'une matière plastique chargée en particules constituées du matériau magnétique.

L'invention concerne également un ensemble de capuchons de fermeture tels que ci-dessus, lesdits capuchons étant disposés dans un contenant de conditionnement comprenant au moins un plateau définissant une pluralité de logements, chaque capuchon de fermeture étant placé dans un logement de manière à ce que le matériau magnétique soit apte à coopérer avec un outil de préhension magnétique.

L'invention concerne enfin un dispositif médical comprenant :
- un capuchon de fermeture tel que précité, et
- un flacon rempli d'un produit pharmaceutique ou chimique, clos hermétiquement par ledit capuchon de fermeture.

### BREVE DESCRIPTION DES FIGURES

D'autres caractéristiques et avantages de l'invention ressortiront de la description détaillée de l'invention qui va suivre en référence aux figures annexées sur lesquelles :
[Fig.1] La [Fig.1] présente un capuchon de fermeture conforme à l'invention, et un flacon, l'ensemble formant un dispositif médical, (a) le capuchon est en position de maintien temporaire sur le flacon, (b) le capuchon est en position verrouillée sur le flacon ;
[Fig.2] La [Fig.2] présente une pluralité de capuchons de fermeture, conformes à l'invention, conditionnés dans un contenant de livraison et adaptés à une préhension magnétique.

### DESCRIPTION DETAILLEE DE L'INVENTION

La présente invention concerne un capuchon de fermeture 2 pour un dispositif médical 10 de type flacon.

Dans le domaine des applications pharmaceutiques, il existe des standards en termes de diamètre intérieur du col 12 du flacon : 13mm et 20mm en sont des exemples.

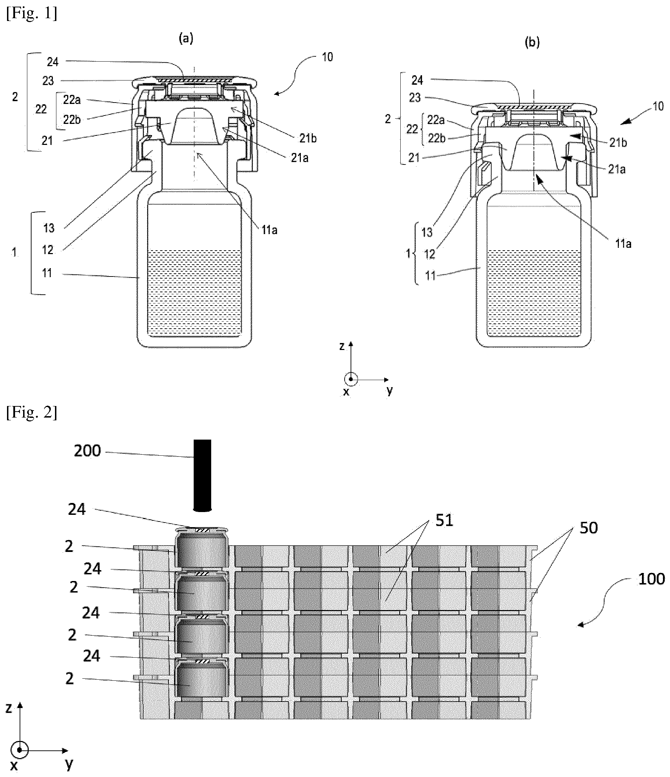

Le capuchon 2 comprend, d'une part, un bouchon 21 en matière élastomère, destiné à être inséré dans le col 12 du flacon 1, après le remplissage de ce dernier avec un produit pharmaceutique ou chimique. Il comprend, d'autre part, une coiffe de verrouillage 22 en matière plastique, entourant le bouchon 21 et destinée à se verrouiller sous la collerette 13 du flacon 1 lorsque le bouchon 21 est inséré dans le col 12. Plus précisément, comme cela est visible sur la [Fig.1](b), dans la position verrouillée du capuchon 2, le pied 21a du bouchon 21 est totalement introduit dans le col 12, et obture hermétiquement l'ouverture 11a du flacon 1. La tête 21b du bouchon 1 reste quant à elle plaquée sur la collerette 13. Un système de clip ou de butée, aménagé sur les parois internes de la coiffe de verrouillage 22 vient s'agripper sous la collerette 13, immobilisant le bouchon 21 dans l'ouverture 11a du flacon 1 et sécurisant ainsi la fermeture du dispositif médical 10.

Sur la [Fig.1](a), le capuchon 2 est uniquement posé sur le flacon 1, en position de maintien temporaire.

La matière plastique constituant la coiffe de verrouillage 22 peut par exemple être du polycarbonate (PC), du polypropylène (PP) ou du poly-téréphtalate de butylène (PBT).

Comme cela est illustré sur la [Fig.1], la coiffe de verrouillage 22 du capuchon 2 peut comprendre un corps externe 22a (ou bague) et une cage 22b (ou muselet), cette dernière étant configurée pour s'emboiter et se verrouiller axialement dans le corps externe 22a. Dans la position de maintien temporaire ([Fig.1] (a)), il est avantageux que des languettes flexibles, ou autre élément de maintien faisant partie de la cage 22b, bloquent le bouchon 21 à l'intérieur de la cage 22b. D'autres languettes flexibles peuvent être aménagées dans la cage 22b pour s'appuyer sur la collerette 13 du flacon 1, lorsque le capuchon 2 est en position de maintien temporaire ; en position de fermeture définitive, ces autres languettes flexibles viennent se verrouiller sous ladite collerette 13 ([Fig.1] (b)).

La coiffe de verrouillage 22 comprend également une capsule 23, au niveau de sa partie supérieure ; en particulier, dans l'exemple de la [Fig.1], la capsule 23 est solidaire du corps externe 22a. Elle peut par exemple être formée d'une des matières plastiques évoquées précédemment pour la coiffe de verrouillage 22.

Cette capsule 23 est destinée à être séparée de manière irréversible du capuchon 2, pour autoriser l'accès à la tête 21b du bouchon 21 : cela intervient lorsque le produit, contenu dans dispositif médical 10 définitivement fermé, doit être prélevé par l'utilisateur final. Alternativement, le capuchon 2 peut être sécable pour permettre à l'utilisateur de le retirer, de manière à accéder au bouchon 21 et à prélever le produit.

Bien sûr, une telle structure de coiffe de verrouillage 22 est donnée à titre d'exemple, et d'autres configurations pourrait être mises en œuvre dans l'objectif de maintenir le bouchon 21 sur le flacon 1, tout en prévoyant un moyen d'accès au produit conservé dans le corps 11 du flacon 1, pour l'utilisateur final.

Le capuchon de fermeture 2 comprend en outre un matériau magnétique 24, destiné à coopérer avec un outil de préhension magnétique, notamment dans les chaines industrielles de remplissage en produits pharmaceutiques ou chimiques.

Selon une première variante, le matériau magnétique forme un aimant permanent, en particulier un aimant néodyme-fer-bore NdFeB ou un aimant en ferrite.

Selon une deuxième variante, le matériau magnétique est ferromagnétique et est choisi en particulier parmi le fer, le cobalt, le nickel, ou des alliages les contenant, ou ferrimagnétique et est choisi en particulier parmi les ferrites grenats et les ferrites spinelles.

Compte tenu du caractère jetable (à usage unique) des capuchons de fermeture 2, cette deuxième variante peut s'avérer plus économique car plusieurs matériaux magnétiques doux sont aisément approvisionnés et peu dispendieux.

Le matériau magnétique 24 peut prendre la forme d'une pièce intégrée à la capsule 23, ou prendre la forme de particules mixées à au moins une matière plastique composant ladite capsule 23.

Avantageusement, le matériau magnétique 24 est solidaire de la coiffe de verrouillage 22 en étant disposé sur ou intégré dans ladite coiffe 22.

Notons qu'il pourrait également être envisageable de disposer le matériau magnétique 24 sur ou de l'intégrer dans le bouchon 21 ; néanmoins, du fait de la proximité directe du bouchon 21 avec le produit pharmaceutique ou chimique contenu dans le flacon 1, il est plus aisé en termes de normes sanitaires, d'associer le matériau magnétique à la coiffe de verrouillage 22.

La capsule 23 est formée d'une matière plastique : une pièce formée dans le matériau magnétique 24 peut donc être intégrée sur ou dans cette dernière. Selon une première option, la pièce en matériau magnétique 24 est complètement ou partiellement surmoulée (encapsulée) dans la capsule 23, lors du moulage de ladite capsule 23. Selon une deuxième option, qui ne fait pas partie de la présente invention, la pièce est collée sur une face supérieure ou une face inférieure de la capsule 23 (dans ce dernier cas, pour se trouver entre la capsule 23 et le corps externe 22a de la coiffe de verrouillage 22), après moulage de la capsule 23. Enfin, dans une troisième option, qui ne fait pas partie de la présente invention, la pièce en matériau magnétique 24 est emmanchée en force dans un logement, réalisé lors du moulage, au niveau de la face supérieure ou de la face inférieure de la capsule 23.

Selon l'une ou l'autre de ces trois options, la pièce en matériau magnétique 24 peut prendre une forme quelconque, pourvu qu'elle soit adaptée à l'association de la pièce sur ou dans la capsule 23. On peut citer notamment les formes de disque, d'anneau ou autre forme polygonale dans le plan (x,y), plan principal de la capsule 23.

Alternativement, la capsule 23 peut être formée d'une matière plastique chargée en particules constituées du matériau magnétique 24.

Lorsque la coiffe de verrouillage 22 ne comprend pas de capsule 23 (le corps externe 22a est alors fermé dans sa partie supérieure), option qui ne fait pas partie de la présente invention, le matériau magnétique 24 est disposé sur ou intégré dans la partie supérieure du corps externe 22a, au centre ou en périphérie, sous une forme quelconque, par exemple disque, anneau ou autre forme polygonale dans le plan (x,y).

Le corps externe 22a est constitué d'une matière plastique : une pièce formée dans le matériau magnétique 24 peut être solidaire dudit corps 22a ou intégrée en tout ou partie dans ce dernier. Les première, deuxième et troisième options précitées peuvent s'appliquer, à savoir :
- la pièce en matériau magnétique 24 est complètement ou partiellement surmoulée dans le corps externe 22a, lors du moulage de celui-ci, ou
- la pièce est collée sur ou sous la partie supérieure du corps externe 22a (dans ce dernier cas, pour se trouver entre le corps externe 22a et la cage 22b), après moulage dudit corps 22a, ou
- la pièce en matériau magnétique 24 est emmanchée en force dans un logement, réalisé lors du moulage, sur ou sous la partie supérieure du corps externe 22a.

Alternativement, le corps externe 22a peut être formé d'une matière plastique chargée en particules constituées du matériau magnétique 24.

Notons que ,selon une option qui ne fait pas partie de la présente invention, le matériau magnétique 24 pourrait également être solidaire de ou intégré en tout ou partie dans la cage 22b, selon les mêmes options que celles décrites ci-dessus.

Le volume et les propriétés magnétiques du matériau magnétique 24 sont dimensionnés de sorte que l'attraction magnétique entre ledit matériau 24 et un outil de préhension magnétique soit suffisante pour soulever et manipuler le capuchon de fermeture 2 dans une chaine industrielle, par exemple au cours d'un procédé de remplissage et de fermeture de flacons 1 pour dispositifs médicaux 10.

Le capuchon de fermeture 2 conforme à l'invention est compatible avec un outil de préhension magnétique 200, tel qu'illustré sur la [Fig.2]. Un tel outil peut prendre différentes formes mais comprend a minima une extrémité munie d'un aimant ou d'un électro-aimant ou formée en matériau ferromagnétique doux.

Dans le cas d'un électro-aimant, celui-ci est activé au moment de l'extraction du capuchon 2 de son plateau de conditionnement 50 jusqu'à son positionnement sur le flacon 1. Il est ensuite désactivé, une fois le capuchon 2 disposé sur le flacon 1 ([Fig.1] (a)) ou, une fois le capuchon 2 définitivement verrouillé sur le flacon 1 ([Fig.1](b)).

Dans le cas d'un aimant simple placé au bout d'un axe, celui-ci doit être suffisamment fort pour permettre l'extraction et le convoyage du capuchon 2 sans risque de chute, jusqu'au positionnement sur le flacon 1, et suffisamment faible pour qu'une fois le capuchon verrouillé sur le flacon, l'aimant de l'outil le relâche, sans soulever le dispositif médical 10 et sans arracher la capsule 23 amovible quand celle-ci est présente.

Selon l'invention, les capuchons de fermeture 2 peuvent être conditionnés sous forme d'un ensemble 100 de capuchons 2. Pour cela, lesdits capuchons 2 sont disposés dans un contenant de conditionnement (non représenté), qui comprend au moins un plateau 50 définissant une pluralité de logements 51 ([Fig.2]). Chaque capuchon de fermeture 2 est placé dans un logement 51 de manière à ce que le matériau magnétique 24 soit apte à coopérer avec l'outil de préhension magnétique 200.

Lorsque le flacon 1 est rempli du produit pharmaceutique, l'outil de préhension magnétique 200 vient prélever un capuchon de fermeture 2 sur le plateau 50, et le place sur le flacon 1, dans la position de maintien temporaire illustrée en [Fig.1] (a). L'outil de préhension 200 peut ensuite être configuré pour lâcher le capuchon 2, qui sera enfoncé en position de verrouillage sur le flacon 1 par application d'une force d'appui, par exemple par une presse intervenant ultérieurement dans la chaine de remplissage. Alternativement, l'outil de préhension 200 peut également être configuré pour appliquer la force d'appui et verrouiller le capuchon 10 sur le flacon 30, avant de relâcher la prise magnétique.

Le capuchon de fermeture 10 selon la présente invention évite l'aspiration de potentielles vapeurs chimiques, tout en autorisant une préhension fiable et pratique dans une chaine industrielle.

L'invention concerne en outre le dispositif médical 10 illustré en [Fig.1](b), qui comprend un capuchon de fermeture 2 tel que décrit ci-dessus et un flacon 1 rempli d'un produit pharmaceutique ou chimique et clos hermétiquement par ledit capuchon 2.

Optionnellement, le dimensionnement (volume et propriétés magnétiques) du matériau magnétique 24 inclus dans le capuchon 2 peut être défini de manière à permettre le convoyage et la préhension du dispositif médical 10, par exemple, pour disposer ledit dispositif 10, après fermeture du flacon 1, dans un contenant de recondi-tionnement collectif ou pour l'extraire d'un tel contenant au stade de l'utilisation finale.

Bien entendu, l'invention n'est pas limitée aux modes de mise en œuvre et exemples décrits, et on peut y apporter des variantes de réalisation sans sortir du cadre de l'invention tel que défini par les revendications.

## Revendications

1. Capuchon de fermeture (2) pour un dispositif médical (10) de type flacon, comprenant :
- un bouchon (21) élastomère destiné à être inséré dans un col (12) d'un flacon (1) après son remplissage avec un produit pharmaceutique ou chimique,
- une coiffe de verrouillage (22) plastique entourant le bouchon (21) et destinée à se verrouiller sous une collerette (13) du flacon (1) lorsque le bouchon (21) est inséré dans le col (12), **caractérisé en ce que** la coiffe de verrouillage (22) comprend, au niveau d'une partie supérieure, une capsule (23),
- un matériau magnétique (24) intégré en tout ou partie dans la capsule (23) ;
et dans lequel :
- la capsule (23) est formée d'une matière plastique et une pièce, formée dans le matériau magnétique (24), est complètement ou partiellement encapsulée dans la matière plastique de ladite capsule (23), ou
- la capsule (23) est formée d'une matière plastique chargée en particules constituées du matériau magnétique (24).

2. Capuchon de fermeture (2) selon la revendication précédente, dans lequel le matériau magnétique (24) forme un aimant permanent, en particulier un aimant néodyme-fer-bore NdFeB ou un aimant en ferrite.

3. Capuchon de fermeture (2) selon la revendication 1, dans lequel le matériau magnétique (24) est ferromagnétique, choisi en particulier parmi le fer, le cobalt, le nickel, ou des alliages les contenant.

4. Capuchon de fermeture (2) selon la revendication 1, dans lequel le matériau magnétique (24) est ferrimagnétique, choisi en particulier parmi les ferrites grenats ou spinelles.

5. Ensemble (100) de capuchons de fermeture (10) selon l'une des revendications précédentes, lesdits capuchons (10) étant disposés dans un contenant de conditionnement comprenant au moins un plateau (50) définissant une pluralité de logements (51), chaque capuchon de fermeture (2) étant placé dans un logement (51) de manière à ce que le matériau magnétique (24) soit apte à coopérer avec un outil de préhension magnétique (200).

6. Dispositif médical (10) comprenant :
- un capuchon de fermeture (2) selon l'une des revendications 1 à 4, et
- un flacon (1) rempli d'un produit pharmaceutique ou chimique, clos hermétiquement par ledit capuchon de fermeture (2).

## Patentansprüche

1. Verschlusskappe (2) für eine flaschenartige medizinische Vorrichtung (10), umfassend:
- einen elastomeren Stopfen (21), der dazu bestimmt ist, in einen Hals (12) einer Flasche (1) eingesetzt zu werden, nachdem diese mit einem pharmazeutischen oder chemischen Produkt gefüllt wurde,
- eine Kunststoff-Verriegelungskappe (22), die den Stopfen (21) umgibt und dazu bestimmt ist, sich unter einem Flansch (13) der Flasche (1) zu verriegeln, wenn der Stopfen (21) in den Hals (12) eingeführt wird, **dadurch gekennzeichnet, dass** die Verriegelungskappe (22) an einem oberen Abschnitt eine Kapsel (23) umfasst,
- ein magnetisches Material (24), das ganz oder teilweise in die Kapsel (23) integriert ist;
und wobei:
- die Kapsel (23) aus einem Kunststoffmaterial gebildet ist und ein Teil, das aus dem magnetischen Material (24) gebildet ist, vollständig oder teilweise in dem Kunststoffmaterial der Kapsel (23) eingekapselt ist, oder
- die Kapsel (23) aus einem Kunststoffmaterial gebildet wird, das mit Partikeln gefüllt ist, die aus dem magnetischen Material (24) bestehen.

2. Verschlusskappe (2) nach dem vorstehenden Anspruch, wobei das magnetische Material (24) einen Permanentmagneten bildet, insbesondere einen Neodym-Eisen-Bor-Magneten NdFeB oder einen Ferritmagneten.

3. Verschlusskappe (2) nach Anspruch 1, wobei das magnetische Material (24) ferromagnetisch ist, insbesondere ausgewählt aus Eisen, Kobalt, Nickel oder Legierungen, die diese enthalten.

4. Verschlusskappe (2) nach Anspruch 1, wobei das magnetische Material (24) ferrimagnetisch ist, insbesondere ausgewählt aus Granat- oder Spinell-Ferriten.

5. Anordnung (100) von Verschlusskappen (10) nach einem der vorstehenden Ansprüche, wobei die Kappen (10) in einem Verpackungsbehälter angeordnet sind, der mindestens eine Schale (50) umfasst, die eine Vielzahl von Aufnahmen (51) definiert, wobei jede Verschlusskappe (2) in einer Aufnahme (51) so angeordnet ist, dass das magnetische Material (24) in der Lage ist, mit einem magnetischen Greifwerkzeug (200) zusammenzuwirken.

6. Medizinische Vorrichtung (10), umfassend:
- eine Verschlusskappe (2) nach einem der Ansprüche 1 bis 4, und
- eine mit einem pharmazeutischen oder chemischen Produkt gefüllte Flasche (1), die durch die Verschlusskappe (2) luftdicht verschlossen ist.

## Claims

1. Closure cap (2) for a vial-type medical device (10), comprising:
- an elastomeric stopper (21) intended to be inserted into a neck (12) of a vial (1) after filling thereof with a pharmaceutical or chemical product,
- a plastic locking cap (22) surrounding the stopper (21) and intended to be locked under a collar (13) of the vial (1) when the stopper (21) is inserted into the neck (12), **characterised in that** the locking cap (22) comprises, at an upper part, a capsule (23),
- a magnetic material (24) integrated in whole or in part in the capsule (23);
and wherein:
- the capsule (23) is formed of a plastics material and a part, formed in the magnetic material (24), is completely or partially encapsulated in the plastics material of said capsule (23), or
- the capsule (23) is formed of a plastics material loaded with particles made of the magnetic material (24).

2. Closure cap (2) according to the preceding claim, wherein the magnetic material (24) forms a permanent magnet, in particular a neodymium-iron-boron NdFeB magnet or a ferrite magnet.

3. Closure cap (2) according to claim 1, wherein the magnetic material (24) is ferromagnetic, in particular selected from iron, cobalt, nickel, or alloys containing them.

4. Closure cap (2) according to claim 1, wherein the magnetic material (24) is ferrimagnetic, in particular selected from garnet or spinel ferrites.

5. Set (100) of closure caps (10) according to one of the preceding claims, said caps (10) being disposed in a packaging container comprising at least one tray (50) defining a plurality of housings (51), each closure cap (2) being placed in a housing (51) such that the magnetic material (24) is able to cooperate with a magnetic gripping tool (200).

6. Medical device (10) comprising:
- a closure cap (2) according to one of claims 1 to 4, and
- a vial (1) filled with a pharmaceutical or chemical product, hermetically closed by said closure cap (2).
